Europäisches Patentamt

European Patent Office

Office européen des brevets

(11) Publication number: **0 243 265 B1**

## EUROPEAN PATENT SPECIFICATION

(45) Date of publication of patent specification: **16.09.92**   (51) Int. Cl.⁵: **A61B 5/02**

(21) Application number: **87400920.2**

(22) Date of filing: **21.04.87**

(54) Electronic sphygmomanometer.

(30) Priority: **21.04.86 JP 90080/86**
**21.04.86 JP 90081/86**

(43) Date of publication of application:
**28.10.87 Bulletin 87/44**

(45) Publication of the grant of the patent:
**16.09.92 Bulletin 92/38**

(84) Designated Contracting States:
**BE DE FR GB IT**

(56) References cited:
**CH-A- 616 801**
**US-A- 4 290 434**
**US-A- 4 312 359**
**US-A- 4 479 494**

(73) Proprietor: **TERUMO KABUSHIKI KAISHA trading as TERUMO CORPORATION**
**44-1, 2-chome, Hatagaya Shibuya-Ku**
**Tokyo 151(JP)**

(72) Inventor: **Ozawa, Hitoshi**
**1663-3, Fujioka Fuji-shi**
**Shizuoka-ken(JP)**

(74) Representative: **Joly, Jean-Jacques et al**
**CABINET BEAU DE LOMENIE 55, rue d'Amsterdam**
**F-75008 Paris(FR)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

This invention relates to a electronic sphygmomanometer and, more particularly, to an electronic sphygmomanometer in which power consumed due to activation of a plurality of independent circuits and peripheral devices is reduced.

In a conventional electronic sphygmomanometer, measurement processing is performed following introduction of electric power to various independent circuits and peripheral devices by a manual operation. The manual introduction of power to the peripheral devices is a troublesome operation. Moreover, during measurement or the printing out of results, the particular processing is executed while power remains applied to devices or circuits which are not participating. As a result, power consumption is high.

Sphygmomanometers having electronically controlled automated functions are known in the art, as can be found in the examples mentioned below.

US-A-4 290 430 discloses a microprocessor-controlled sphygmomanometer in which the activation of a pressure cuff and the detection of Korotkoff sounds is carried out according to a programmed sequence. The results are shown on an electronic display.

CH-A-616 801 teaches a sphygmomanometer in which an electronic display can be powered separately by a manual switch to save power.

US-A-4 479 494 relates to an electronically adaptive pressure cuff for a sphygmomanometer having, among other things, a time display function.

In particular, an electronic sphygmomanometer having a timekeeping function is adapted to display date and time information when blood pressure is measured. Since the time, for example, is displayed in a display window constantly regardless of whether the window cover is open or closed, the amount of power consumed by the display is significant. Though it has been contemplated to display the time information in the display window only when the cover is open, power continues to be supplied to the control unit (namely a CPU and its peripheral circuitry) during the time that a switch or the like for starting blood pressure measurement is in the ON state. For this reason, the reduction in power consumption is still inadequate.

Accordingly, an object of the invention is to provide an electronic sphygmomanometer in which power consumption is curtailed by executing blood pressure processing upon introducing power only to peripheral elements required at the time of such processing.

Another object of the invention is to provide an electronic sphygmomanometer in which power can be supplied to peripherals automatically to simplify and facilitate operation.

According to the present invention, the foregoing objects are attained by providing an electronic sphygmomanometer comprising an electronic sphygmomanometer body which includes display means for displaying time information and measured blood pressure information, timekeeping means supplied with power at all times for outputting a pulse signal of a predetermined duration at fixed intervals determined by the minimum time unit that can be displayed by said display means and for holding time information, processing means for executing blood pressure measurement, and cover means for covering at least said display means; said sphygmomanometer body further including first power supply means for supplying said display means with power to indicate a display enable state in response to detecting that said cover means is open, and second power supply means for supplying power to said processing means at least during the period during which the pulse is outputted by the timekeeping means when said display means is placed in a display enable state, and said processing means including first control means for controlling said second power supply means, inhibiting any interruptions to the power supply, when said pulse signal is outputted from said timekeeping means, read-in means for reading in time information from said timekeeping means after said second power supply means is activated by said first control means, output means for outputting the time information to said display means whereafter said first control means deactivates said second power supply means so as to cut off the power to said processing means, and second control means for controlling said second power supply means, so as to permit interruptions after said time information is outputted by said output means. This arrangement makes it possible to minimize power consumed by the time display.

The cover means may consist in a cap covering the upper face of the electronic sphygmomanometer body. Power consumed by the time display can be minimized even when the cap is open, and the cap makes it possible to protect at least the upper face of the electronic sphygmomanometer body.

In another embodiment, the cover means is a case for accommodating the electronic sphygmomanometer body. Thus, power consumed by the time display can be minimized and the case makes it possible to protect the electronic sphygmomanometer.

The period of the pulse signal outputted by the timekeeping means may be one minute. This makes it possible to update the least significant digit of the time display.

The width of the pulse signal outputted by the timekeeping means preferably corresponds to at least a period of time from the leading edge of the pulse signal until the processing means becomes operational. This assures that the processing means will operate in a reliable manner.

The electronic sphygmomanometer further includes a memory for storing plural items of blood pressure information whenever a measurement is performed, the memory being supplied with power at all times to hold this blood pressure information. Data indicative of measured values therefore will not be erased.

Other features and advantages of the sphygmomanometer according to the invention will be apparent from the following description taken in conjunction with the accompanying drawings, wherein:

Fig. 1 is a block diagram of an electronic sphygmomanometer embodying the present invention;

Figs. 2(a) and (b) are flowcharts illustrating operation of the electronic sphygmomanometer embodying the present invention;

Fig. 3(a) is a sectional view of the electronic sphygmomanometer embodying the present invention; and

Figs. 3(b) and (c) are views illustrating the relationship between a cover and a switch.

An embodiment of the present invention will now be described in detail with reference to the accompanying drawings.

In this embodiment, the electronic sphygmomanometer is powered by a battery, by way of example.

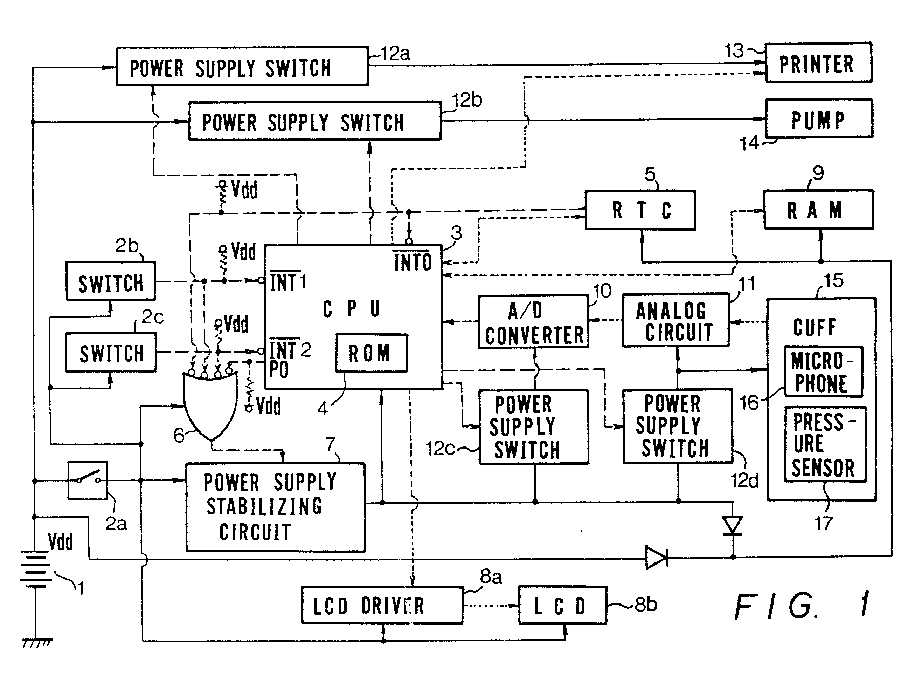

In the block diagram of Fig. 1, the solid lines indicate power supply lines, the dashed lines represent control lines between a CPU 3 and a switch 2a, described below, and the dotted lines represent data exchange lines, primarily a data bus. The circuits and peripheral devices of the overall block diagram are supplied with electric power by a battery 1. The battery 1 is connected to the switch 2a which discriminates whether e.g. a cover of the electronic sphygmomanometer is in place. If the cover has been removed from the electronic sphygmomanometer, the switch 2a closes to provide a power supply stabilizing circuit 7, switches 2b, 2c, and LCD drivers 8a, 8b described below, with power from the battery 1.

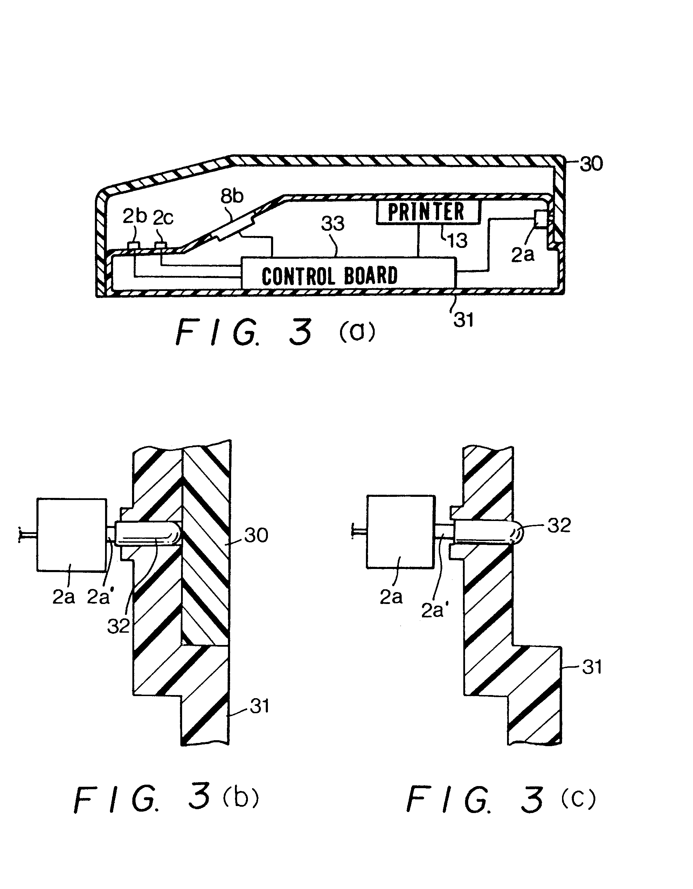

As shown in (a) of Fig. 3, the switch 2a is situated at e.g. the rear of the electronic sphygmomanometer body, designated by numeral 31. Disposed within the electronic sphygmomanometer body is a control board 33 comprising an IC, which includes the CPU 3, and various LSIs. The switch 2a has a head 2a' which, in the state shown in (b) of Fig. 3, is caused to recede to the left of the body 31 by a cover 30 acting upon a button 32 the base end of which is in abutting contact with the head 2a'. In this state the switch is off, i.e. open. Fig. 3(c) depicts the state which prevails when the cover 30 is removed from the body 31. Since removing the cover removes the leftward urging force from the buttom 32, the latter is thrust rightward beyond the face of the body 31 by the restoring force of the head 2a', whereby the switch 2a is turned on or closed. There is no particular limitation upon the configurations and positions of the cover 30 and switch 2a as long as the switch 2a is capable of discriminating whether the cover 30 is open or closed. It is permissible to connect the cover 30 to the electronic sphygmomanometer body by a hinge. It is also possible to place the entirety of the electronic sphygmomanometer body in a case, rather than provide the body with the cover 30. In other words, since it will suffice to adopt an arrangement in which it can be detected when the electronic sphygmomanometer is not in use, the easiest approach is to provide the cover 30 for covering at least the display, which is an LCD 8b. The cover 30 is useful in protecting at least the upper face of the electronic sphygmomanometer when it is carried, while the case is advantageous in that it protects the entirety of the electronic sphygmomanometer.

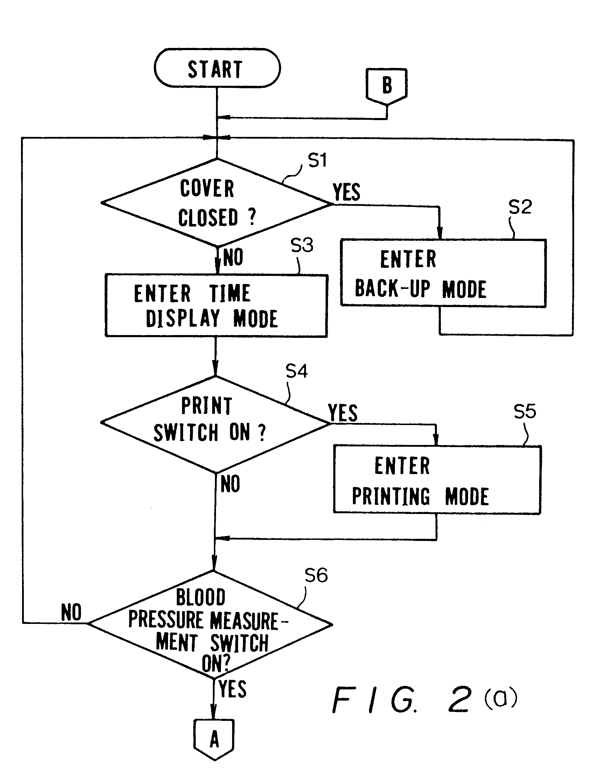

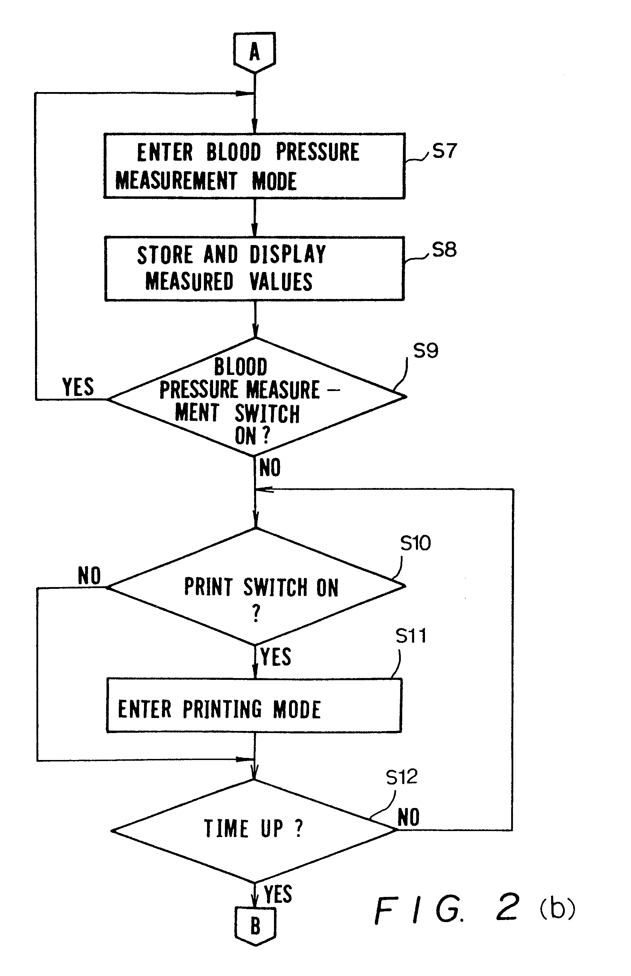

The switch 2b is for commanding the print-out of measured blood pressure values and information relating to the date and time of measurement. The switch 2c is for commanding the start of blood pressure measurement. The CPU 3 controls the various circuits and peripheral devices and includes a ROM 4 storing a control program illustrated by the flowchart of Fig. 2, described below. A real-time clock (hereinafter referred to as an "RTC") 5 is supplied with electrical power at all times and functions in the same manner as a clock, generating an interrupt signal every minute for application to the CPU 3. A gate 6 is actuated when the switch 2a closes. When at least one of the inputs thereto is logical "0", the gate 6 outputs logical "1". The output of gate 6 is applied to the power supply stabilizing circuit 7, which is for supplying stabilized power to various circuits and peripheral devices when the output of gate 6 is "1". Numeral 8a denotes an LCD driver which drives the LCD 8b for the display. The LCD driver 8a is supplied with power when the switch 2a is ON, namely when the cover 30 has been removed. A RAM 9 stores various data relating to measured values as well as information relating to the date and time of measurement. Like the RTC 5, the RAM 9 is supplied with power at all times for data back-up purposes. At the time of a blood pressure measurement, an A/D converter 10 receives an analog Korotkoff signal from a microphone 16 and an analog pressure level signal from a pressure sensor 17, which are provided in a pressure cuff 15, and converts these signals into digital signals applied to the CPU 3. An analog circuit 11 comprises an amplifier and the like for amplifying the Korotkoff signal and pressure level signal from the pressure cuff 15. Power supply switches 12a, 12b, 12c, 12d are controlled by the CPU 3 to control the supply of power to a printer

3

13, pump 14, the A/D converter 10 and the analog circuit 11, respectively. Note that the control lines of the RTC 5 and switches 2b, 2c as well as an output terminal PO of the CPU 3 are pulled up to the logic level "Vdd" so as to attain a high level, namely logical "1", when the switch 2a is closed.

If the results of blood pressure measurement and the like are to be printed out at the end of measurement in the electronic sphygmomanometer having the foregoing construction, power need not be supplied to the pump 14. Conversely, when performing processing for printing or the like, it is unnecessary to drive or operate the analog circuit 11, the A/D converter 10 and a peripheral device such as the pump 14.

Thus, it will be appreciated that power should be supplied to each peripheral device and circuit as it is needed for a particular operation. The various processing stages and the circuits or peripheral devices required therefor are summerized generally in the following table. In the illustrated embodiment, the processing stages are described as being four in number, namely a back-up mode, time display mode, blood pressure measurement mode and printing mode, though these modes can be broken down further if desired. In the table, the symbol "x" under a mode means that the indicated device or circuit is not supplied with power when the mode of operation is executed. The symbol "Δ" means that the device or circuit is supplied with power sometimes when the particular mode of operation is executed. The symbol "0" indicates that the device or circuit is supplied with power at all times.

TABLE I

|  | Back-Up Mode | Time Display Mode | Blood Pressure Meas. Mode | Printing Mode |
|---|---|---|---|---|
| CPU | x | Δ | O | O |
| RTC & RAM | O | O | O | O |
| LCD & LCD Driver | x | O | O | O |
| Analog Circuit & |  |  |  |  |
| A/D Converter | x | x | O | x |
| Power Supply Stabilizing Circuit | x | O | O | O |
| Pump | x | x | O | x |
| Printer | x | x | x | O |

Operation in each of these modes will now be described.

The back-up mode refers to the state in which the cover 30 is placed on the sphygmomanometer, namely in which power is being supplied to the RTC 5, serving as the clock, and to the back-up RAM 9. At this time, the RTC 5 provides the CPU 3 with an interrupt signal in the form of a negative signal having a predetermined duration. The signal is applied to the CPU 3 e.g. every minute, which is the least significant digit of the time display. This signal is also connected to the gate 6. Meanwhile, in order for the RAM 9 to hold data which is the result of previous measurement, the RAM is supplied with power at all times. In other words, the RAM is powered constantly for back-up purposes. In this mode, power is not supplied to the CPU 3, by way of example.

The time display mode refers to the state in which the cover 30 is removed from the electronic sphygmomanometer, on which occasion time is displayed by the LCD 8b, which constitutes the display means of the electronic sphygmomanometer. In this mode, the switch 2a is closed to supply power from battery 1 to the LCD 8b, LCD driver 8a, gate 6 and switches 2b, 2c. Since the output of gate 6 is logical "0" at this time, the power supply stabilizing circuit 7 does output the supply voltage, so that the CPU 3 naturally remains without power. However, the RTC 5 is always operating, as the block diagram of Fig.1 shows, and therefore is constantly providing the CPU 3 and the gate 6 every minute with the negative signal having the predetermined duration. In response to this signal applied thereto, the gate 6 outputs a logical "1" signal to the power supply stabilizing circuit 7, which is responsive to supply power to the CPU 3 and an IC, not shown, which is necessary for operating the CPU 3. When the CPU 3 receives power, the output PO therefor is immediately made "0" during the time that the output signal of the RTC 5 is at the "0" level. The "0" output PO prevents power from being cut off to the CPU 3 so that the latter may read in the current time from the RTC 5 and write the time data into the LCD driver 8a to latch the same. The driver 8a causes the LCD 8b to display the time data. When the above processing ends, the CPU 3 sends its output terminal PO to logical "1", whereby power to the CPU 3 per se is cut off. Note that even though power to the CPU 3 is cut off, the LCD driver 8a latches the time data so that the data continues to be displayed by the LCD 8b. Since the foregoing operation is performed every minute, in the time display mode the CPU 3 is supplied with power every minute rather than at all times, thus making it possible to reduce the power consumed by the CPU. For this reason, the CPU 3 is assigned the "△" mark in the "Time Display Mode" column of the table. In this mode, power need be supplied only to the RTC 5, RAM 9, LCD 8b, LCD driver 8a and the stabilizing circuit 7. Ordinarily, the arrangement is such that the CPU 3 is disabled immediately after the stabilizing circuit 7 supplies it with power. The reason is that a reset signal is supplied to the CPU 3 for several blocks so that the CPU 3 can execute the program reliably from the normal address. Accordingly, it is required that the pulse width of the pulse signal outputted by the RTC 5 be greater than this time interval at least.

In the blood pressure measurement mode, the output of the gate 6 goes to logical "1" in response to closure of the switch 2c, and power is supplied to the CPU 3, just as in the time display mode. The CPU 3 responds first by placing logical "0" at its output terminal PO to prevent power from being cut off to the CPU. The CPU 3 then applies signals to the power supply switches 12b - 12d, as a result of which the switches are closed to supply power to the pump 14, A/D converter 10 and analog circuit 11, respectively. Thereafter, in accordance with a well-known processing procedure, pressure inside the cuff 15 is raised, a signal is received from the pressure sensor 17, operation of the pump 14 is halted when internal cuff pressure attains a predetermined level, air within the cuff 13 is discharged via a constant-rate discharge valve, not shown, thereby depressurizing the cuff, during which time pulse rate is measured, as well as systolic and diastolic blood pressure on the basis of Korotkoff sounds from the microphone 16. The results of blood pressure measurement, namely the systolic and diastolic blood pressure and pulse rate, are displayed on the LCD 8b by activating the LCD driver 8a. The results are also stored in the RAM 9. Next, the CPU 3 determines whether the switch 2b has been pressed for a prescribed period of time. If it has not, the CPU 3 removes signals from the power supply switches 12a - 12d and then places logical "1" at its output terminal PO, whereby power is cut off from the CPU 3 and peripheral circuits. Strictly speaking, the period of time during which power is supplied to the pump 14 is not equal to the duration of blood pressure measurement. It will suffice if the power is supplied until cuff pressure reaches a predetermined value. In other words, the duration of power supply to the pump 14 extends from the moment air begins to be fed into the cuff 15 until the predetermined value is attained.

The printing mode refers to outputting the previous item of measurement value data, which has been stored in RAM 9, or a currently measured blood pressure value and pulse rate to the printer 13. When the CPU 3 senses that the switch 2b has been pressed, it first places logical "0" at its output terminal PO to prevent a cut-off in power, just as in the blood pressure measurement mode. The CPU 3 then turns on the power supply switch 12a to introduce power to the printer 13. Thereafter, the data stored in RAM 9 are outputted to the printer 13. When it is detected that the processing means has outputted to the printer 13 all information that is to be printed, end printing is completed, the CPU 3 again raises its output PO to logical "1" to remove power from the CPU.

This completes the description of each mode of processing. The flow of a series of these processing steps will now be described with reference to the' flowchart of Figs. 2(a) and (b). Each mode mentioned in the flowchart is as set forth above.

Step S1 of the flowchart calls for a determination as to whether the cover 30 of the electronic sphygmomanometer has been closed. If the answer is YES, then the sphygmomanometer operates in accordance with the back-up mode at S2. If the cover 30 has not been closed, on the other hand, the time display mode is established at step S3 in response to the signal by the RTC 5 every minute. This causes the LCD 8b to display the time. The program then proceeds to step S4, at which it is determined whether the print switch 2b is ON. If this switch is found to be ON, the program proceeds to a step S5, at which the printing mode is established and the CPU 3 reads in the previous measurement data from the RAM 9 and outputs it to the printer 13. Next, it is determined at step S6 whether the blood pressure measurement switch 2c is ON. If the answer is NO, the program returns to step S1. If the switch 2c is ON, then the program proceeds to step S7, at which the blood pressure measurement mode is established. Accordingly, systolic and diastolic blood pressure and pulse rate are measured, and the resulting data are displayed by the LCD 8b via the LCD driver 8a at step S8, and currently stored in the RAM 9 together with the data of time and date when the measurement has been performed at the same step. This is followed by step S9, at which the status of blood pressure measurement switch 2c is sensed. If the switch is ON, then processing is repeated from step S7 onward; if the switch is OFF, the status of the print switch 2b is sensed at step S10. When the switch 2b is found to be ON, the data resulting from the measurement performed at step S7 are outputted to the printer (step S11). When the switch 2b is OFF, on the other hand, the operation for sensing the status of switch 2b is repeated for a predetermined period of time. If the switch remains OFF longer than the predetermined time period, as a result of the test performed at step S12, the program returns to step S1.

Thus, in accordance with the present embodiment as described above, power is supplied only to the circuits and peripheral devices required for each type of processing, thereby enabling a reduction in power consumption. When a battery is employed as the power source, the service life thereof can be prolonged.

By automating the delivery and removal of power to and from the peripheral devices, less labor is involved in operating the sphygmomanometer. This arrangement also assures that power to the peripherals will not be left on inadvertently. Moreover, the fact that the measurement data are stored in the constantly powered RAM assured that the data will not be erased accidentally.

If the switch for starting blood pressure measurement or the switch for starting printing processing is not pressed again within a predetermined period of time after blood pressure measurement or measured value print-out processing, power is cut off from the CPU proper. This makes it possible to reduce the amount of power consumed.

Power is supplied to the display means when the cover 30 is opened, in which state power is delivered to the CPU every minute, namely at the time updating interval, to display and update the time. This improves the driving ratio of the CPU with respect to time to minimize the power consumed thereby.

Though the invention has been described with regard to an embodiment in which a battery serves as the power supply, the invention is not so limited. If the invention adopts a commercial AC power supply as the power supply, the amount of power consumed can be greatly reduced.

In the illustrated embodiment, power is supplied to the CPU in response to a pulse signal generated every minute. However, if the information relating to blood pressure measurement need only be obtained once per hour, an arrangement can be adopted in which the pulse signal is generated every hour. It is also possible to set the pulse generation period to any other value.

**Claims**

1. An electronic sphygmomanometer comprising an electronic sphygmomanometer body which includes display means (8a, 8b) for displaying time information and measured blood pressure information, timekeeping means (5) supplied with power at all times for outputting a pulse signal of a predetermined duration at fixed intervals determined by the minimum time unit that can be displayed by said display means and for holding time information, processing means (3) for executing blood pressure measurement, and cover means (30) for covering at least said display means (8a, 8b); wherein

   - said sphygmomanometer body further includes first power supply means (1) for supplying said display means (8a, 8b) with power to indicate a display enable state in response to detecting that said cover means (30 is open, and second power supply means (7) for supplying power to said processing means at least during the period during which the pulse is outputted by the timekeeping means (5) when said display means (8a, 8b) is placed in a display enable state, and
   - said processing means (3) includes first control means (ouput Po, 6) for controlling said second power supply, inhibiting any interruptions to the power supply, when said pulse is outputted from said timekeeping means (5), read-in means for reading in time information from said timekeeping means (5) after said second power supply means is activated by said first control means, output means for outputting the time information to said display means (8a, 8b) whereafter said first control means (Po, 6) deactivates said second power supply means (7) so as to cut off the power to said processing means (3), and second control means (2b, 2c, 6) for controlling said second power supply means (7), so as to permit interruptions after said time information is outputted by said output means.

2. A sphygmomanometer according to claim 1, characterized in that said cover means (30) is a cap covering an upper face of the sphygmomanometer body.

3. A sphygmomanometer according to claim 1, characterized in that the cover means is a case for accommodating the sphygmomanometer body.

4. A sphygmomanometer according to any one of claims 1 to 3, characterized in that the interval of the pulse signal ouputted by said timekeeping means (5) is one minute.

5. A sphygmomanometer according to any one of claims 1 to 4, characterized in that the pulse signal outputted by said timekeeping means has a width corresponding to at least a period of time from a leading edge of the pulse signal until said processing means becomes operational.

6. A sphygmomanometer according to any one of claims 1 to 5, characterized by a memory (9) for storing plural items of blood pressure information whenever a measurement is performed, said memory (9) being supplied with power at all times to hold this blood pressure information.

## Patentansprüche

1. Elektronischer Blutdruckmesser, umfassend einen elektronischen Blutdruckmesser-Körper mit einer Anzeigeeinheit (8a, 8b) zum Anzeigen von Zeitinformation und gemessener Blutdruckinformation, einer ständig mit Strom gespeisten Zeithalteeinheit (5) zum Ausgeben eines Impulssignals einer vorbestimmten Dauer in festen Intervallen, die durch die kleinste Zeiteinheit bestimmt werden, welche von der Anzeigeeinheit angezeigt werden kann, und zum Halten von Zeitinformation, einer Verarbeitungseinheit (3) zum Ausführen einer Blutdruckmessung und einer Abdeck- oder Deckeleinheit (30) zum Abdecken zumindest der Anzeigeeinheit, bei welchem
   - der Blutdruckmesser-Körper ferner eine erste Stromversorgungseinheit (1) zum Speisen der Anzeigeeinheit mit Strom für das Anzeigen eines Anzeigefreigabezustands in Abhängigkeit von der Feststellung, daß die Deckeleinheit (30) offen ist, und eine zweite Stromversorgung (7) zum Zuspeisen von Strom zur Verarbeitungseinheit zumindest während der Zeitspanne oder Periode, in welcher der Impuls von der Zeithalteeinheit (5) ausgegeben wird, wenn die Anzeigeeinheit (8a, 8b) in einen Anzeigefreigabezustand gesetzt ist, aufweist und
   - die Verarbeitungseinheit (3) eine erste Steuereinheit (Ausgang PO, 6) zum Steuern der zweiten Stromversorgung zwecks Unterbindung etwaiger Unterbrechungen (zu) der Stromversorgung, wenn der Impuls von der Zeithalteeinheit (5) ausgegeben wird, eine Einleseeinheit zum Einlesen von Zeitinformation von der Zeithalteeinheit (5) nach dem Aktivieren der zweiten Stromversorgung durch die erste Steuereinheit, eine Ausgabeeinheit zum Ausgeben der Zeitinformation zur Anzeigeeinheit (8a, 8b), worauf die erste Steuereinheit (PO, 6) die zweite Stromversorgung (7) deaktiviert, um die Stromzufuhr zur Verarbeitungseinheit (3) abzuschalten, und eine zweite Steuereinheit (2b, 2c, 6) zum Steuern der zweiten Stromversorgung (7) zwecks Ermöglichung von Unterbrechungen nach der Ausgabe der Zeitinformation durch die Ausgabeeinheit aufweist.

2. Blutdruckmesser nach Anspruch 1, dadurch gekennzeichnet, daß die Deckeleinheit (30) eine eine Oberseite des Blutdruckmesser-Körpers abdeckende Kappe ist.

3. Blutdruckmesser nach Anspruch 1, dadurch gekennzeichnet, daß die Deckeleinheit ein Gehäuse zur Aufnahme oder Unterbringung des Blutdruckmesser-Körpers ist.

4. Blutdruckmesser nach einem der Ansprüche 1 bis 3, dadurch gekennzeichnet, daß das Intervall des durch die Zeithalteeinheit (5) ausgegebenen Impulssignals eine Minute ist.

5. Blutdruckmesser nach einem der Ansprüche 1 bis 4, dadurch gekennzeichnet, daß das von der Zeithalteeinheit ausgegebene Impulssignal eine Breite entsprechend zumindest einer Periode von einer Vorderflanke des Impulssignals bis zu dem Punkt, zu dem die Verarbeitungseinheit betriebsbereit wird, aufweist.

6. Blutdruckmesser nach einem der Ansprüche 1 bis 5, gekennzeichnet durch einen Speicher (9) zum Speichern einer Anzahl von Einzelheiten oder Posten der Blutdruckinformation bei jedesmaliger Durchführung einer Messung, wobei der Speicher (9) ständig mit Strom gespeist wird, um diese Blutdruckinformation zu halten.

## Revendications

1. Sphygmomanomètre électronique comprenant un corps de sphygmomanomètre électronique qui comporte un moyen d'affichage (8a, 8b) servant à afficher une information horaire et une information se rapportant à la tension artérielle mesurée, un moyen (5) de conservation de temps électriquement alimenté en permanence qui sert à délivrer un signal d'impulsion d'une durée prédéterminée à des intervalles fixes déterminés par l'unité de temps minimal qui peut être affichée par ledit moyen d'affichage et à conserver l'information horaire, un moyen de traitement (3) servant à exécuter la mesure de tension artérielle, et un moyen de revêtement protecteur (30) qui recouvre au moins ledit moyen d'affichage (8a, 8b), où

ledit corps de sphygmomanomètre comporte en outre un premier moyen d'alimentation électrique (1) qui alimente électriquement ledit moyen d'affichage (8a, 8b) pour indiquer un état de validation d'affichage en réponse à la détection du fait que ledit moyen de revêtement protecteur (30) est ouvert, et un deuxième moyen d'alimentation électrique (7) qui alimente électriquement ledit moyen de traitement au moins pendant la durée pendant laquelle l'impulsion est délivrée par le moyen de conservation du temps (5) lorsque ledit moyen d'affichage (8a, 8b) est placé dans un état de validation d'affichage, et

ledit moyen de traitement (3) comporte un premier moyen de commande (sortie P0, 6) servant à commander ladite deuxième alimentation électrique, en empêchant toute interruption de l'alimentation électrique, lorsque ladite impulsion est délivrée par ledit moyen de conservation de temps (5), un moyen de lecture servant à lire l'information horaire dans ledit moyen de conservation de temps (5) après que ledit deuxième moyen d'alimentation électrique a été activé par ledit premier moyen de commande, un moyen de sortie servant à délivrer l'information horaire audit moyen d'affichage (8a, 8b), après quoi ledit premier moyen de commande (PO, 6) désactive ledit deuxième moyen d'alimentation électrique (7) de façon à couper l'alimentation électrique dudit moyen de traitement (3), et un deuxième moyen de commande (2b, 2c, 6) servant à commander ledit deuxième moyen d'alimentation électrique (7) de façon à autoriser les interruptions après que ladite information horaire a été délivrée par ledit moyen de sortie.

2. Sphygmomanomètre selon la revendication 1, caractérisé en ce que ledit moyen de revêtement protecteur (30) est un couvercle couvrant une face supérieure du corps de sphygmomanomètre.

3. Sphygmomanomètre selon la revendication 1, caractérisé en ce que le moyen de revêtement protecteur est un boîtier destiné à loger le corps de sphygmomanomètre.

4. Sphygmomanomètre selon l'une quelconque des revendications 1 à 3, caractérisé en ce que l'intervalle du signal d'impulsion délivré par ledit moyen de conservation de temps (5) est de 1 min.

5. Sphygmomanomètre selon l'une quelconque des revendications 1 à 4, caractérisé en ce que le signal d'impulsion délivré par ledit moyen de conservation de temps possède une largeur qui correspond au moins à la durée qui va du flanc antérieur du signal d'impulsion jusqu'au moment où ledit moyen de traitement devient opérationnel.

6. Sphygmomanomètre selon l'une quelconque des revendications 1 à 5, caractérisé par une mémoire (9) servant à emmagasiner plusieurs articles d'information de tension artérielle à chaque fois qu'une mesure est effectuée, ladite mémoire (9) étant électriquement alimentée en permanence pour maintenir cette information de tension artérielle.

FIG. 1

EP 0 243 265 B1

FIG. 2 (a)

FIG. 2 (b)

2b 2c

8b

PRINTER

13

33

2a

CONTROL BOARD

30

31

F I G. 3 (a)

2a 2a'

32

30

31

F I G. 3 (b)

2a

2a'

32

31

F I G. 3 (c)